# EUROPEAN PATENT APPLICATION

(11) **EP 4 159 755 A1**
(43) Date of publication of application: **05.04.2023**
(21) Application number: 21814546.4
(22) Date of filing: 21.05.2021
(51) Int. Cl.: C07K 16/00, C07K 17/00, C12P 19/32, C12N 15/115, C12Q 1/48, C12Q 1/66, C12N 9/12, G01N 33/543

(54) **METHOD FOR DETECTING TARGET MOLECULE IN SPECIMEN, AND TARGET MOLECULE DETECTION KIT**

(30) Priority: 25.05.2020 JP 2020090398
(71) Applicant: Yokogawa Electric Corporation, Musashino-shi Tokyo 180-8750 (JP)
(72) Inventor: AKIYOSHI Ryutaro, Musashino-shi, Tokyo 180-8750 (JP)
(74) Representative: Winter, Brandl - Partnerschaft mbB
(86) International application number: PCT/JP2021/019390
(87) International publication number: WO 2021/241446

(57) **Abstract**

A detection method of a target molecule in a specimen includes a (target molecule)-(labeled binding molecule)-(capturing molecule) complex forming step of reacting the target molecule in the specimen, a capturing molecule that binds to the target molecule, and a labeled binding molecule that binds to the target molecule and which is labeled with an enzyme that catalyzes a reaction to produce ATP, to form a complex formed of the target molecule, the capturing molecule, and the labeled binding molecule, a step of removing the labeled binding molecules which did not bind to the target molecule, an ATP production step of producing ATP by reacting the (target molecule)-(labeled binding molecule)-(capturing molecule) complex with a substrate of the enzyme that catalyzes a reaction to produce ATP, an ATP amplification step of amplifying the produced ATP, and an ATP detection step of detecting the amplified ATP.

## Description

### TECHNICAL FIELD

The present invention relates to a detection method of a target molecule in a specimen and a detection kit for a target molecule.

### BACKGROUND ART

Immunoassay methods are widely used as methods for detecting target objects, such as viruses in human body fluids and allergens in foods. Immunochromatography is an immunoassay method, in which an application is made of a property (capillary phenomenon) of a specimen to flow slowly across a cellulose membrane while dissolving a reagent, and is applied to pregnancy diagnosis, influenza tests, and the like.

However, immunochromatography has an advantage in that measurement is extremely easy to perform, but uses a method of determination based on coloration and thus has low sensitivity. In the determination of influenza and the like, it is not possible to detect viruses in a case where the amount of virus in the specimen is low immediately after the onset of illness or the like and re-measurement may be necessary after a period of time for the amount of virus to increase sufficiently.

ELISA (Enzyme-Linked ImmunoSorbent Assay) is a detection method of a target molecule in a specimen using combinations of various antigen-antibody reactions by incorporating an enzyme-labeled antigen or antibody into a reaction system and detecting enzyme activity and is able to detect target molecules with high sensitivity, in comparison with methods for visually determining coloration by metallic colloids, which are typically adopted in immunochromatography. In ELISA, using substrates that change absorbance spectra according to an enzyme reaction (colorimetric method), substrates that emit fluorescent signals (fluorescence method), substrates that emit light (chemiluminescence method, bioluminescence method), and the like, it is possible to quantify enzyme activity with a measurement apparatus such as a plate reader or a luminometer and detect and quantify the target molecule.

On the other hand, there is known a method for quantifying ATP by using an enzyme that catalyzes a reaction to produce ATP, making the produced ATP emit luminescence using luciferase, and measuring the amount of luminescence produced (Patent Document 1). In addition, there is known a method in which ATP in a specimen is amplified and the amplified ATP is quantified using a bioluminescence method (Patent Document 2). However, all of these quantification methods are methods for quantifying molecules used in an ATP conversion reaction, such as ATP, and are not immunoassay methods that detect target molecules not involved in an ATP conversion reaction in a specimen by using an enzyme that catalyzes a reaction to produce ATP as a labeled enzyme.

As an immunoassay method that carries out detection using an enzyme that catalyzes a reaction to produce ATP as a labeled enzyme, Non-Patent Document 1 discloses an immunoassay method for a target molecule in which acetate kinase or pyruvate phosphate dikinase is used as a labeled enzyme. However, this method is only able to obtain luminescence signals on the basis of the amount of ATP produced, which depends solely on the metabolic turnover rate of acetate kinase or pyruvate phosphate dikinase, and is not able to detect trace amounts of ATP. In addition, this method is not suitable for commercial use in detection kits or the like because the initial reaction is carried out by supplying ATP through acetate kinase using ADP, which is easily degraded.

### Citation List

### Patent Documents

[Patent Document 1] Japanese Unexamined Patent Application, First Publication No. H9-234099
[Patent Document 2] Japanese Unexamined Patent Application, First Publication No. 2001-299390

### Non-Patent Document

[Non-Patent Document 1] Anal Sci. 2003 Jan; 19(1): 105-9.

### DISCLOSURE OF INVENTION

### Technical Problem

In immunoassay methods such as immunochromatography and ELISA, the sensitivity is determined by the antibody labeling substance and the detection method thereof. Among immunoassay methods, the bioluminescence method, which is able to detect molecules with the highest sensitivity, is capable of detecting molecules to an extent of approximately 10⁻²⁰ mol (thousands of molecules) in one assay; however, even when using the bioluminescence method, it is necessary for the specimen to include several thousand molecules or more of the target molecules per assay and if the number of molecules is less than the above number, it is not possible to detect the target molecules.

Accordingly, the object of the present invention is to provide a detection method of a target molecule in a specimen and a target molecule detection kit, which are able to carry out measurement even in a case where the number of target molecules in the specimen is at or below a detection limit and thus is not measurable by an immunoassay method of the related art.

### [Solution to Problem]

In order to achieve the object described above, the present invention adopts the following configurations.
[1] A detection method of a target molecule in a specimen, the method including:
   step (1): a complex formed of a target molecule, a labeled binding molecule, and a capturing molecule (referred to below as a (target molecule)-(labeled binding molecule)-(capturing molecule) complex) forming step of reacting the target molecule in the specimen, a capturing molecule that binds to the target molecule, and a labeled binding molecule that binds to the target molecule and which is labeled with an enzyme that catalyzes a reaction to produce ATP, to form a (target molecule)-(labeled binding molecule)-(capturing molecule) complex;
   step (2): a step of removing the labeled binding molecules which did not bind to the target molecule in the step (1);
   step (3): an ATP production step of producing ATP by reacting the (target molecule)-(labeled binding molecule)-(capturing molecule) complex with a substrate of the enzyme that catalyzes a reaction to produce ATP;
   step (4): an ATP amplification step of amplifying the ATP produced in the step (3); and
   step (5): an ATP detection step of detecting the ATP amplified in the step (4).
[2] The detection method according to [1], further including a step of removing ATP in the specimen in advance before forming the (target molecule)-(labeled binding molecule)-(capturing molecule) complex in the step (1).
[3] The detection method according to [1] or [2], in which the capturing molecule is an antibody or antibody fragment, or an aptamer that specifically binds to the target molecule.
[4] The detection method according to any one of [1] to [3], in which the labeled binding molecule is an antibody or antibody fragment, of an aptamer that specifically binds to the target molecule and which is labeled with the enzyme that catalyzes a reaction to produce ATP.
[5] The detection method according to any one of [1] to [4], in which the enzyme that catalyzes a reaction to produce ATP is pyruvate phosphate dikinase, and the substrates of the enzyme that catalyzes a reaction to produce ATP are phosphoenolpyruvate, pyrophosphate, and AMP.
[6] The detection method according to any one of [1] to [4], in which the enzyme that catalyzes a reaction to produce ATP is acetyl-CoA synthetase, and the substrates of the enzyme that catalyzes a reaction to produce ATP are AMP, pyrophosphate, and acetyl-CoA.
[7] The detection method according to any one of [1] to [4], in which the enzyme that catalyzes a reaction to produce ATP is ATP-sulfurylase, and the substrates of the enzyme that catalyzes a reaction to produce ATP are adenosine 5'-phosphosulfate and pyrophosphate.
[8] The detection method according to any one of [1] to [4], in which the enzyme that catalyzes a reaction to produce ATP is a type II polyphosphate kinase, and the substrates of the enzyme that catalyzes a reaction to produce ATP are AMP and polyphosphate.
[9] The detection method according to any one of [1] to [8], in which the amplification of the ATP is performed using adenylate kinase and pyruvate kinase in the step (4).
[10] The detection method according to any one of [1] to [8], in which the amplification of the ATP is performed using adenylate kinase and polyphosphate kinase in the step (4).
[11] The detection method according to any one of [1] to [8], in which the amplification of the ATP is performed using adenylate kinase and acetate kinase in the step (4).
[12] The detection method according to any one of [1] to [11], in which the detection of the ATP is performed using luciferase in the step (5).
[13] A detection kit for a target molecule in a specimen, the kit including an insoluble carrier on which a capturing molecule that binds to the target molecule is immobilized, a labeled binding molecule that binds to the target molecule and which is labeled with an enzyme that catalyzes a reaction to produce ATP, a substrate of the enzyme that catalyzes a reaction to produce ATP, a reagent that amplifies ATP, and a reagent that detects ATP.
[14] The detection kit according to [13], in which the capturing molecule is an antibody of antibody fragment, or an aptamer that specifically binds to the target molecule.
[15] The detection kit according to [13] or [14], in which the labeled binding molecule is an antibody or antibody fragment, or an aptamer that specifically binds to the target molecule and which is labeled with the enzyme that catalyzes a reaction to produce ATP.
[16] The detection kit according to any one of [13] to [15], in which the enzyme that catalyzes a reaction to produce ATP is pyruvate phosphate dikinase, and the substrates of the enzyme that catalyzes a reaction to produce ATP are phosphoenolpyruvate, pyrophosphate, and AMP.
[17] The detection kit according to any one of [13] to [15], in which the enzyme that catalyzes a reaction to produce ATP is acetyl-CoA synthetase, and the substrates of the enzyme that catalyzes a reaction to produce ATP are AMP, pyrophosphate, and acetyl-CoA.
[18] The detection kit according to any one of [13] to [15], in which the enzyme that catalyzes a reaction to produce ATP is ATP-sulfurylase, and the substrates of the enzyme that catalyzes a reaction to produce ATP are adenosine 5'-phosphosulfate and pyrophosphate.
[19] The detection kit according to any one of [13] to [15], in which the enzyme that catalyzes a reaction to produce ATP is a type 11 polyphosphate kinase, and the substrates of the enzyme that catalyzes a reaction to produce ATP are AMP and polyphosphate.
[20] The detection kit according to any one of [13] to [19], in which the reagent that amplifies ATP include AMP, phosphoenolpyruvate, magnesium ions, adenylate kinase, and pyruvate kinase.
[21] The detection kit according to any one of [13] to [19], in which the reagent that amplifies ATP includes AMP, polyphosphate, magnesium ions, adenylate kinase, and polyphosphate kinase.
[22] The detection kit according to any one of [13] to [19], in which the reagent that amplifies ATP includes AMP, acetyl phosphate, magnesium ions, adenylate kinase, and acetate kinase.
[23] The detection kit according to any one of [13] to [22], in which the reagent that detects ATP includes D-luciferin and luciferase.

### Advantageous Effects of Invention

According to the detection method of a target molecule in a specimen of the present invention, it is possible to detect a target molecule in a specimen even in a case where the number of target molecules in the specimen is at or below a detection limit and thus is not measurable by an immunoassay method of the related art. In addition, according to the detection kit for a target molecule in a specimen of the present invention, there is provided a detection kit for a target molecule capable of detecting a target molecule in a specimen even in a case where the number of target molecules in the specimen is at or below a detection limit and thus is not measurable by an immunoassay kit of the related art.

### BRIEF DESCRIPTION OF DRAWINGS

FIG. 1 is a diagram showing a state in which a target molecule 10 in a specimen, a capturing molecule 20 that binds to the target molecule 10 and which is immobilized on an insoluble carrier 40, and a labeled binding molecule 30 labeled with an enzyme that catalyzes a reaction to produce ATP are reacted and a (target molecule)-(labeled binding molecule)-(capturing molecule) complex is formed on the insoluble carrier 40.
FIG. 2 is a diagram showing an example of an operating procedure of the detection method of a target molecule in a case where an ATP amplification step and an ATP detection step are performed separately.
FIG. 3 is a diagram showing an example of an operating procedure of the detection method of a target molecule in a case where the ATP amplification step and the ATP detection step are performed simultaneously.
FIG. 4 is a diagram showing an example of an operating procedure of a method for quantifying a target molecule in a case where the ATP amplification step and the ATP detection step are performed simultaneously.
FIG. 5A is a diagram showing a side surface of an example configuration in immunochromatography using the detection method of a target molecule of the present embodiment.
FIG. 5B is a diagram showing the top surface of an example configuration in immunochromatography using the detection method of a target molecule of the present embodiment.

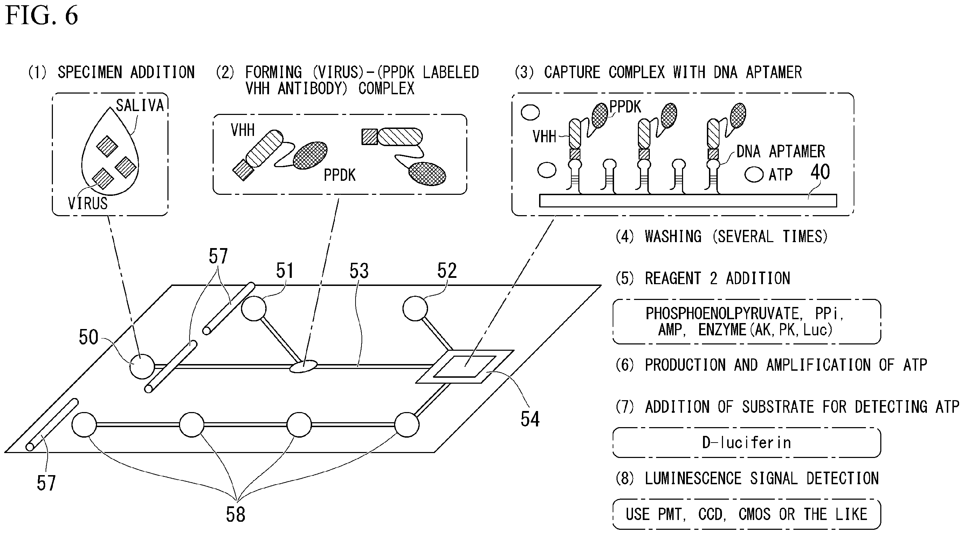

FIG. 6 is a diagram showing an example of an example configuration and an operation in immunochromatography, in which, using a cartridge in which a liquid is fed by externally applying force to an elastic container with a roller, a virus as the target molecule 10, a DNA aptamer as a capturing antibody 20 that binds to the target molecule 10, a pyruvate phosphate dikinase-labeled heavy chain variable region antibody as a labeled binding substance 30 labeled with an enzyme that catalyzes a reaction to produce ATP, adenylate kinase and pyruvate kinase as enzymes for amplifying ATP, luciferase as an enzyme for detecting ATP, a cycloolefin polymer (COP) as the insoluble carrier 40, are used.
FIG. 7 is a graph showing the luminescence intensity of samples with sample numbers 1 to 7 of Example 1.
FIG. 8 is a graph showing the luminescence intensity of a negative control (a case where ATP amplification was performed and a case where ATP amplification was not performed), in a case where a PPDK-labeled anti-BSA-VHH antibody is used in combination with a reagent that amplifies ATP and in a case where the PPDK-labeled anti-BSA-VHH antibody is used alone in Example 2.
FIG. 9 is a graph showing, in a case where a PPDK-labeled anti-BSA-VHH antibody is used in combination with a reagent that amplifies ATP and in a case where the PPDK-labeled anti-BSA-VHH antibody is used alone in Example 2, the luminescence intensity subtracted from each of the luminescence intensity of the negative control in a case where ATP amplification was performed and the luminescence intensity of the negative control in a case where ATP amplification was not performed, as backgrounds.

### BEST MODE FOR CARRYING OUT THE INVENTION

The detection method of a target molecule in a specimen of the present invention includes
step (1): a (target molecule)-(labeled binding molecule)-(capturing molecule) complex forming step of reacting the target molecule in the specimen, a capturing molecule that binds to the target molecule, and a labeled binding molecule that binds to the target molecule and which is labeled with an enzyme that catalyzes a reaction to produce ATP, and forming a (target molecule)-(labeled binding molecule)-(capturing molecule) complex,
step (2): a step of removing the labeled binding molecules which did not bind to the target molecule in the step (1),
step (3): an ATP production step of producing ATP by reacting the (target molecule)-(labeled binding molecule)-(capturing molecule) complex with a substrate of the enzyme that catalyzes a reaction to produce ATP,
step (4): an ATP amplification step of amplifying the ATP produced in the step (3), and
step (5): an ATP detection step of detecting the ATP amplified in the step (4).

A description will be given below of embodiments of the detection method of a target molecule in a specimen of the present invention.

In the following description, unless otherwise specified, a capturing molecule and a labeled binding molecule mean a capturing molecule that binds to a target molecule and a labeled binding molecule that binds to a target molecule and which is labeled with an enzyme that catalyzes a reaction to produce ATP, respectively.

### [Step (1)]

Step (1) is a (target molecule)-(labeled binding molecule)-(capturing molecule) complex forming step of forming a (target molecule)-(labeled binding molecule)-(capturing molecule) complex by reacting a target molecule in a specimen, a capturing molecule, and a labeled binding molecule.

In step (1), the method for reacting the target molecule in the specimen, the capturing molecule, and the labeled binding molecule may be a method for simultaneously reacting the target molecule in the specimen, the capturing molecule, and the labeled binding molecule or may be a method for reacting the target molecule and the capturing molecule, forming a (target molecule)-(capturing molecule) complex formed of the target molecule and the capturing molecule, and then carrying out a reaction with the labeled binding molecule. In addition, the method may also be a method in which the target molecule and the labeled binding molecule are reacted, a (target molecule)-(labeled binding molecule) complex formed of the target molecule and the labeled binding molecule is formed, and then a reaction is carried out with the capturing molecule.

The capturing molecule may be immobilized or not immobilized on an insoluble carrier, but immobilization is preferable.

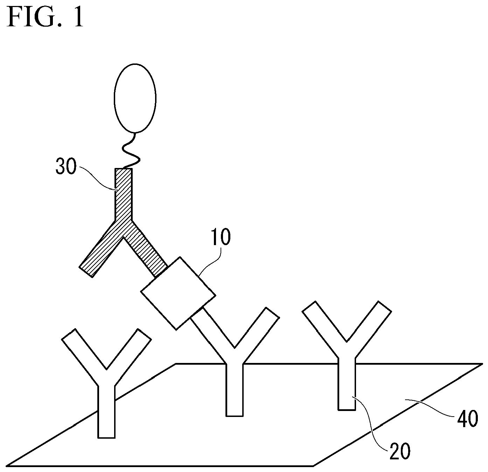

FIG. 1 shows a state in which, in the step (1), the target molecule 10 in the specimen, the capturing molecules 20 immobilized on the insoluble carrier 40, and the labeled binding molecule 30 are reacted and a (target molecule)-(labeled binding molecule)-(capturing molecule) complex is formed on the insoluble carrier 40. In a case where the capturing molecule 20 is immobilized on the insoluble carrier 40, in step (2) described below, washing the insoluble carrier 40 with a washing solution makes it possible to easily separate the (target molecule)-(labeled binding molecule)-(capturing molecule) complex formed in step (1) from unreacted components (components derived from the specimen, the labeled binding molecules 30 that did not bind to the target molecules 10, and the like). Examples of washing solutions include phosphate buffered saline (also referred to below as PBS), PBS containing a surfactant, an aqueous medium described below, and the like. Examples of the surfactants include non-ionic surfactants such as Tween 20, or the like.

The insoluble carrier 40 is not particularly limited other than being capable of immobilizing the capturing molecule 20. Examples of preferable materials for the insoluble carrier 40 include polymer materials such as polystyrene, polycarbonate, polyvinyltoluene, polypropylene, polyethylene, polyvinyl chloride, nylon, polymethacrylate, gelatin, agarose, cellulose, nitrocellulose, cellulose acetate, cellulose acetate, cyclo-olefin polymers, and polyethylene terephthalate, glass, ceramics, magnetic particles and metals, and the like and cellulose acetate, cyclo-olefin polymers, and the like which do not adsorb proteins and are capable of suppressing nonspecific binding of the labeled binding molecules 30 to the insoluble carrier are preferable. Preferable forms of insoluble carriers include tubes, beads, plates, substrates, fine particles such as latex, sticks, and the like.

As methods for immobilizing the capturing molecule 20 on the insoluble carrier 40, it is possible to use known methods such as methods using physical bonding, methods using chemical bonding, combinations thereof, or the like. Examples of physical bonding include electrostatic bonding, hydrogen bonding, hydrophobic bonding, and the like. Examples of chemical bonding include covalent bonding, coordination bonding, and the like. For example, in a case where a substrate made of a cyclopolyolefin-based polymer is used as the insoluble carrier 40, exemplary examples thereof include a method of physical adsorption and immobilization by adding a solution of the capturing molecule 20 to the substrate and carrying out incubation at 4°C to 30°C for 1 hour to 1 day.

The capturing molecule 20 may be directly immobilized on the insoluble carrier 40 or may be indirectly immobilized on the insoluble carrier 40. Examples of indirect immobilization methods include a method in which a solution of the biotinylated capturing molecule 20 is added to the insoluble carrier 40 on which avidin is immobilized and the capturing molecule 20 is immobilized on the insoluble carrier 40 via specific binding of biotin and avidin. In addition, an antibody that specifically binds to the capturing molecule 20 may be immobilized on the insoluble carrier 40 and the capturing molecule 20 may be immobilized on the insoluble carrier 40 via this antibody. Alternatively, the capturing molecule 20 may be immobilized on the insoluble carrier 40 by covalent bonding via a linker. Examples of linkers include molecules or the like capable of covalently bonding to both the functional group of the capturing molecule 20 and the functional group retained on the surface of the insoluble carrier 40. As the molecules, molecules having, in the same molecule, a first reaction active group capable of reacting with the functional group of the capturing molecule 20 and a second reaction active group capable of reacting with the functional group retained on the surface of the insoluble carrier 40 are preferable. Among the above, molecules in which the first reaction active group and second reaction active group are different groups are particularly preferable. Examples of the functional group of the capturing molecule 20 and functional group retained on the surface of the insoluble carrier 40 include carboxyl groups and amino groups, glycidyl groups, sulfhydryl groups, hydroxyl groups, amide groups, imino groups, N-hydroxysuccinyl groups, maleimide groups, and the like. Examples of active reactive groups in linkers include groups of allyl azides, carbodiimides, hydrazides, aldehydes, hydroxymethylphosphine, imido esters, isocyanates, maleimides, N-hydroxysuccinimide (NHS) esters, pentafluorophenyl (PFP) esters, solarenes, pyridyl disulfides, vinyl sulfones, and the like.

In the detection method in the present embodiment, the specimen is not particularly limited and examples thereof include body fluids such as blood, blood cells, serum, plasma, spinal fluid, urine, sweat, saliva, amniotic fluid, and pancreatic juice, tissues, and the like of mammals such as humans, foods and extracts from foods, cell culture media and the like of animal cells, plant cells, insect cells, microbial cells, and the like, washing solutions for medical devices and the like, or the like. The specimen may be collected from the object of the detection, or may be subjected to a treatment such as dilution or concentration normally performed on collected specimens. In addition, the specimens used in the detection method of the present embodiment may be collected or prepared at the time of implementing the detection method of the present embodiment or may be collected or prepared and stored in advance.

In the detection method in the present embodiment, the target molecule 10 is not particularly limited other than being the molecule which is the target of the detection in the detection method of the present embodiment and being capable of being detected by the detection method of the present embodiment and examples thereof include proteins, nucleic acids, lipids, vitamins, polysaccharides, low molecular weight compounds, and the like. Examples of nucleic acids include DNA, RNA, and the like. In addition, examples of proteins include enzymes, hormones, various peptides, and the like.

In the detection method in the present embodiment, the capturing molecule 20 is not particularly limited other than being a molecule capable of specifically binding to the target molecule 10 in the specimen and examples thereof include an antibody or antibody fragment that specifically binds to the target molecule 10, an aptamer that specifically binds to the target molecule 10, or the like. As antibodies, it is possible to use both polyclonal antibodies and monoclonal antibodies, but monoclonal antibodies are preferable. Examples of antibody fragments include F(ab')₂, F(ab)₂, Fab', Fab, Fv, scFv, mutants thereof, fusion proteins of fusion peptides containing antibody portions, and the like.

As the antibody fragments, it is also possible to use heavy chain variable region antibodies of camelid antibodies (also referred to below as VHH antibodies). It is possible for VHH antibodies to bind to antigens solely in the heavy chain variable region. In addition, it is also possible to use VHH antibodies in tandem and tandemization makes it possible to improve the binding affinity to the antigen which is the target molecule and to reduce non-specific binding to molecules other than the antigen.

It is possible to produce these antibodies and antibody fragments by known methods.

Aptamers that specifically bind to target molecules in the specimen may be nucleic acid aptamers such as DNA aptamers, RNA aptamers, or may be peptide aptamers. It is possible to produce the nucleic acid aptamers using a known method such as the in vitro selection method or the SELEX method. In addition, the nucleic acid aptamers may also be molecularly modified with 2'-fluoropyrimidine, PEG chains, or the like and it is possible to lengthen the half-life of the nucleic acid aptamer by the molecular modification.

In the detection method in the present embodiment, the enzyme that catalyzes a reaction to produce ATP is not particularly limited other than being an enzyme capable of catalyzing the reaction to produce ATP and examples thereof include pyruvate phosphate dikinase (also referred to below as PPDK), acetyl-CoA synthetase, ATP-sulfurylase, type II polyphosphate kinase, and the like and PPDK is preferable.

As shown in the following formula, PPDK is a known enzyme, also called pyruvate orthophosphate dikinase, which interacts with AMP, phosphoenol-pyruvate and pyrophosphate (PPi) in the presence of magnesium ions as cofactors to catalyze a reaction which creates ATP, pyruvate, and phosphate (Pi) and which also catalyzes the reverse reaction.

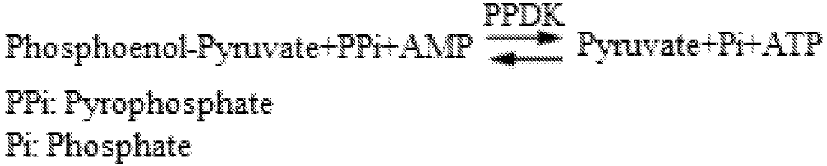

In the detection method in the present embodiment, the labeled binding molecule 30 is not particularly limited other than being a molecule that binds to the target molecule 10 and which is labeled with an enzyme that catalyzes a reaction to produce ATP and examples thereof include antibodies, antibody fragments, aptamers or the like that specifically bind to the target molecule 10 and which are labeled with an enzyme that catalyzes a reaction to produce ATP. The labeled binding molecule 30 may bind to the target molecule 10 at a different portion than the capturing molecule 20 or may bind to the target molecule 10 at the same portion as the capturing molecule 20.

Examples of the antibodies, antibody fragments, and aptamers for the labeled binding molecule 30 include the antibodies, antibody fragments, and aptamers for the capturing molecule 20 described above. The combination of the labeled binding molecule 30 and the capturing molecule 20 may be any of antibodies with other antibodies, a combination of antibodies and antibody fragments, a combination of antibodies and aptamers, a combination of antibody fragments and aptamers, and the like.

It is possible to perform the labeling of the labeled binding molecule 30 by a reaction that produces a covalent bond, either via a linker or not, between a functional group of the molecule that binds to the target molecule 10 and a functional group of an enzyme that catalyzes a reaction to produce ATP. Examples of the functional groups include carboxyl groups or amino groups, glycidyl groups, sulfhydryl groups, hydroxyl groups, amide groups, imino groups, hydroxysuccinyl ester groups, maleimide groups, isothiocyanate groups, and the like. It is possible to perform condensation reactions between these functional groups.

Examples of binding methods without linkers include a method using a carbodiimide compounds such as EDC. In such a case, it is also possible to use active esters such as NHS or a derivative thereof. Condensation reactions between isothiocyanate and amino groups do not require other reagents and proceed simply by mixing under neutral to slightly alkaline conditions and are thus preferable.

Examples of linkers include linkers having, in the molecule, both functional groups of a functional group that reacts with the functional group in the molecule binding to the target molecule 10 and a functional group that reacts with the functional group of the enzyme that catalyzes a reaction to produce ATP. As linkers having both functional groups in the molecule, molecules having, in the same molecule, a first functional group capable of reacting with the amino acid residue of the molecule that binds to the target molecule 10 and a second functional group capable of reacting with the functional group of the enzyme that catalyzes a reaction to produce ATP are preferable. Among the above, a molecule in which the first functional group and the second functional group are different groups is particularly preferable. Examples of the functional groups of the linker include the functional groups described above.

In addition, in a case where the molecule that binds to the target molecule 10 is a protein, such as an antibody or antibody fragment, it is also possible to produce the molecule that binds to the target molecule 10 and the enzyme that catalyzes a reaction to produce ATP as a single fusion protein. In the fusion protein, a linker peptide of several residues to several tens of residues may be inserted between the molecule that binds to the target molecule 10 and the enzyme that catalyzes a reaction to produce ATP. In addition, in the fusion protein, the enzyme that catalyzes a reaction to produce ATP may be fused to either the N-terminal side or the C-terminal side of the molecule that binds the target molecule 10.

In step (1), before forming the (target molecule)-(labeled binding molecule)-(capturing molecule) complex, ATP in the specimen is preferably removed in advance. By removing ATP in the specimen in advance before forming the (target molecule)-(labeled binding molecule)-(capturing molecule) complex in step (1), in step (4) described below, it is possible to suppress the amplification of the ATP and the generation of false positives due to the ATP present in the specimen and to accurately detect the target molecules 10 in the specimen.

The method for removing ATP in the specimen in advance before forming the (target molecule)-(labeled binding molecule)-(capturing molecule) complex is not particularly limited other than being a method with which it is possible to remove ATP from the specimen and examples thereof include a method for removing ATP by washing, a method for degrading and removing ATP through an reaction with an enzyme that degrades ATP, such as adenosine phosphate deaminase, apyrase, or hexokinase.

### [Step (2)]

Step (2) is a step of removing the labeled binding molecules 30 that did not bind to the target molecules 10 in step (1). By removing the labeled binding molecules 30 that did not bind to the target molecule in step (1), it is possible to suppress the generation of false positives and to accurately detect the target molecule. In addition, as well as the labeled binding molecules 30 that did not bind to the target molecule in step (1), it is also possible to remove components derived from the specimen other than the target molecule 10.

In step (2), the method for removing the labeled binding molecules 30 that did not bind to the target molecule is not particularly limited and examples thereof include washing, centrifugation, and the like, although washing is preferable. Examples thereof include a method for washing the insoluble carrier, on which the (capturing molecule)-(target molecule)-(labeled binding molecule) complex is immobilized, using a washing solution. Examples of washing solutions include PBS, PBS containing a surfactant, aqueous media as described below, and the like. Examples of surfactants include non-ionic surfactants such as Tween 20 or the like.

### [Step (3)]

Step (3) is an ATP production step of producing ATP by reacting the (target molecule)-(labeled binding molecule)-(capturing molecule) complex with a substrate of the enzyme that catalyzes a reaction to produce ATP.

It is possible to appropriately determine the substrate of the enzyme that catalyzes a reaction to produce ATP according to the enzyme that catalyzes a reaction to produce ATP. For example, in a case where the enzyme that catalyzes a reaction to produce ATP is PPDK, examples thereof include phosphoenolpyruvate, pyrophosphate, and AMP. In a case where the enzyme that catalyzes a reaction to produce ATP is acetyl-CoA synthetase, examples thereof include AMP, pyrophosphate, and acetyl-CoA. In a case where the enzyme that catalyzes a reaction to produce ATP is ATP-sulfurylase, examples thereof include adenosine 5'-phosphosulfate and pyrophosphate. In a case where the enzyme that catalyzes a reaction to produce ATP is a type 11 polyphosphate kinase, examples thereof include AMP and polyphosphate. The reaction between the substrate of the enzyme that catalyzes a reaction to produce ATP and the enzyme that catalyzes a reaction to produce ATP is performed in the presence of cofactors such as magnesium ions, according to the enzyme to be used.

In a case where a labeled binding molecule labeled with PPDK is used as the enzyme that catalyzes a reaction to produce ATP, phosphoenolpyruvate, pyrophosphate (PPi), and AMP are reacted with the (target molecule)-(labeled binding molecule)-(capturing molecule) complex produced in the step (1) in the presence of magnesium ions as cofactors to produce pyruvate, phosphate (Pi), and ATP. The produced ATP is provided for the ATP amplification step which is the next step (4).

### [Step (4)]

Step (4) is an ATP amplification step in which the ATP produced in the step (3) is amplified. Although the method for amplifying ATP is not particularly limited, a method using a reagent that amplifies ATP is preferable. Examples of reagents for amplifying ATP include enzymes that amplify ATP and substrates thereof. In a case where enzymes that amplify ATP and substrates thereof are used, the reagent that amplifies ATP includes cofactors necessary for the enzyme reaction. Examples of the enzyme that amplifies ATP include a combination of adenylate kinase (referred to below as AK) and pyruvate kinase (referred to below as PK), a combination of AK and polyphosphate kinase, a combination of AK and creatine kinase, a combination of AK and acetate kinase, and the like, but the combination of AK and PK is preferable.

It is possible to appropriately determine the substrate and cofactor for the enzyme that amplifies ATP according to the enzyme that amplifies ATP. For example, in a case where the enzyme that amplifies ATP is a combination of AK and PK, examples of substrates of the enzyme that amplifies ATP include AMP and phosphoenolpyruvate and examples of cofactors include magnesium ions. In a case where the enzyme that amplifies ATP is a combination of AK and polyphosphate kinase, examples of substrates of the enzyme that amplifies ATP include AMP and polyphosphate and examples of cofactors include magnesium ions. In a case where the enzyme that amplifies ATP is a combination of AK and creatinine kinase, examples of substrates of the enzyme that amplifies ATP include AMP and creatine phosphate and examples of cofactors include magnesium ions. In a case where the enzyme that amplifies ATP is a combination of AK and acetate kinase, examples of substrates of the enzyme that amplifies ATP include AMP and acetyl phosphate and examples of cofactors include magnesium ions.

The following reaction formula shows a method for amplifying ATP using a combination of AK and PK.

First Cycle
Adenylate kinase (AK)
ATP + AMP AK 2ADP
Pyruvate kinase (PK)
2ADP + 2 phosphoenol-pyruvate PK 2 pyruvate + 2ATP
Second Cycle
Adenylate kinase (AK)
2ATP + 2AMP AK 4ADP
Pyruvate kinase (PK)
4ADP + 4 phosphoenol-pyruvate PK 4 pyruvate + 4ATP
Nth Cycle
2^n ATP

In the method for amplifying ATP using AK and PK, in the presence of magnesium ions as cofactors, using AK, two molecules of ADP are produced from one molecule of ATP produced in step (3) and one molecule of AMP. Next, in the presence of magnesium ions as a cofactor, using PK, two molecules of pyruvate and two molecules of ATP are produced from the two produced molecules of ADP and two molecules of phosphoenolpyruvate (first cycle). Once more, through the reaction system using AK and PK described above, 4 molecules of ATP are produced from the two molecules of ATP produced in the first cycle (second cycle). By repeating this cycle n times, ATP is amplified 2ⁿ times in the nth cycle.

Step (4) may be performed simultaneously with the step (3). In a case where step (4) is performed simultaneously with step (3), in step (3), by adding a substrate of the enzyme that catalyzes a reaction to produce ATP and a reagent that amplifies ATP to the (target molecule)-(labeled binding molecule)-(capturing molecule) complex, it is possible to perform ATP production and ATP amplification simultaneously. For example, in a case where the enzyme that catalyzes a reaction to produce ATP is PPDK and ATP is amplified using AK and PK, by adding phosphoenolpyruvate, pyrophosphate (PPi), AMP, AK, PK, and magnesium ions to the (target molecule)-(labeled binding molecule)-(capturing molecule) complex, it is possible to perform the production and amplification of ATP simultaneously.

### [Step (5)]

Step (5) is an ATP detection step in which the ATP amplified in the step (4) is detected. Although the method for detecting ATP is not particularly limited, a method using a reagent that detects ATP is preferable. Examples of reagents for detecting ATP include enzymes that react with ATP to produce dye or luminescence and substrates thereof, enzymes that react with ATP to produce hydrogen peroxide and substrates thereof, and the like.

Examples of methods using reagents for detecting ATP include a method for measuring the luminescence produced by ATP and D-luciferin using luciferase, a method using glucokinase and acetate kinase in combination as enzymes to detect ATP in order to amplify the reaction that produces glucose-6-phosphate from ATP and glucose and further combining glucose-6-phosphate dehydrogenase and diaphorase to perform a visible coloration reaction (Japanese Unexamined Patent Application, First Publication No. 2003-225098), a method using hexokinase and pyruvate kinase in combination to amplify the reaction to produce glucose 6-phosphate from ATP and D-glucose, and carrying out quantification using chemiluminescence using 1-methoxy 5-phenazine methylsulfate and isoluminol (Japanese Unexamined Patent Application, First Publication No. S64-23900), a method for adding nucleoside phosphorylase and xanthine oxidase to ATP and quantifying the hydrogen peroxide produced in the reaction (Japanese Unexamined Patent Application, First Publication No. S63-11848), a method in which an ATP-degrading enzyme acts on ATP and molybdenum blue produced by a reaction of the produced phosphate and molybdenum is measured (Japanese Unexamined Patent Application, First Publication No. H4-360700 and the like), a method in which nicotinic acid amide mononucleotide and adenylyltransferase act on ATP and the produced NAD is quantified by performing a redox reaction system using NAD as a coenzyme and a coenzyme cycling reaction using reduced NAD as a coenzyme (Japanese Unexamined Patent Application, First Publication No. S59-166099), and the like, but a method for measuring the luminescence produced by ATP and D-luciferin using luciferase is preferable. In addition, as a method for detecting ATP, it is also possible to use commercially available kits for detecting ATP levels using colorimetry or fluorescence (for example, ATP Colorimetric/Fluorometric Assay Kit, manufactured by BioVision).

Luciferase is used as the enzyme for detecting ATP and the reaction formula for the luminescence generated by ATP amplified in step (4) and D-luciferin is shown below.

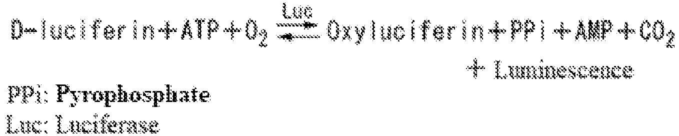

As luciferases, for example, it is possible to use beetle luciferase derived from fireflies such as Photinus pyralis, click beetles, or the like, recombinant beetle luciferase, mutants of beetle luciferase with heat resistance and/or chemical resistance imparted thereto, or any luciferase that reacts with ATP, luciferin, molecular oxygen, and magnesium or any divalent cation as a cofactor to produce luminescence. In addition to D-luciferin, it is also possible to use luciferin analogs such as aminoluciferin and AkaLumine as substrates of luciferase.

The ATP detection reaction in step (5) has a slower reaction speed than the ATP production reaction in step (3) and the ATP amplification reaction in step (4), thus, it is possible to detect ATP even if step (5) is performed simultaneously with step (3) and step (4). In a case where step (3), step (4), and step (5) are performed simultaneously, by adding the substrate of the enzyme that catalyzes a reaction to produce ATP to the (target molecule)-(labeled binding molecule)-(capturing molecule) complex in step (3) and simultaneously adding a reagent that amplifies ATP and a reagent that detects ATP thereto, it is possible to perform the production, amplification, and detection of ATP simultaneously. For example, in a case where the enzyme that catalyzes a reaction to produce ATP is PPDK, AK and PK are used to amplify ATP, and luciferase is used to detect ATP, by adding phosphoenolpyruvate, pyrophosphate, AMP, AK, PK, D-luciferin, and luciferase and magnesium ions as cofactors to the (target molecule)-(labeled binding molecule)-(capturing molecule) complex in step (3), it is possible to perform the production, amplification, and detection of ATP simultaneously.

By performing step (1) to step (5) using a known concentration of the target molecule instead of the specimen, it is possible to create a calibration curve representing the relationship between the concentration of the target molecule and the signal in the ATP detection step, to perform step (1) to step (5) using the specimen, and to quantify the concentration of the target molecule in the specimen from the measurement values of the signals detected in step (5) and the created calibration curve.

In addition, it is also possible to quantify the concentration of the target molecule in the specimen by performing step (1) to step (5) using a known concentration of the target molecule, measuring the change over time in signal intensity from the signal generation in the ATP detection step to create a signal intensity increase curve, performing step (1) to step (5) using the specimen, and performing curve fitting with the signal intensity increase curve in step (5).

In the detection method of a target molecule of the present embodiment, step (1) and step (3) to step (5) are preferably performed in an aqueous medium. Examples of the aqueous medium include deionized water, distilled water, a buffer solution, and the like and a buffer solution is preferable. The buffers used in the preparation of buffer solutions are not particularly limited other than having a buffering ability and examples thereof include pH 1 to 11 lactate buffers, citrate buffers, acetate buffers, succinate buffers, phthalate buffers, phosphate buffers, triethanolamine buffers, diethanolamine buffers, lysine buffers, barbiturate buffers, imidazole buffers, malate buffers, oxalate buffers, glycine buffers, borate buffers, carbonate buffers, glycine buffers, tris buffers, Good's buffers, and the like.

The concentration of the buffer solution is not particularly limited as long as the concentration is suitable for detection of the target molecule, but 0.001 mol/L to 2.0 mol/L is preferable, 0.005 mol/L to 1.0 mol/L is more preferable, and 0.01 mol/L to 0.1 mol/L is particularly preferable.

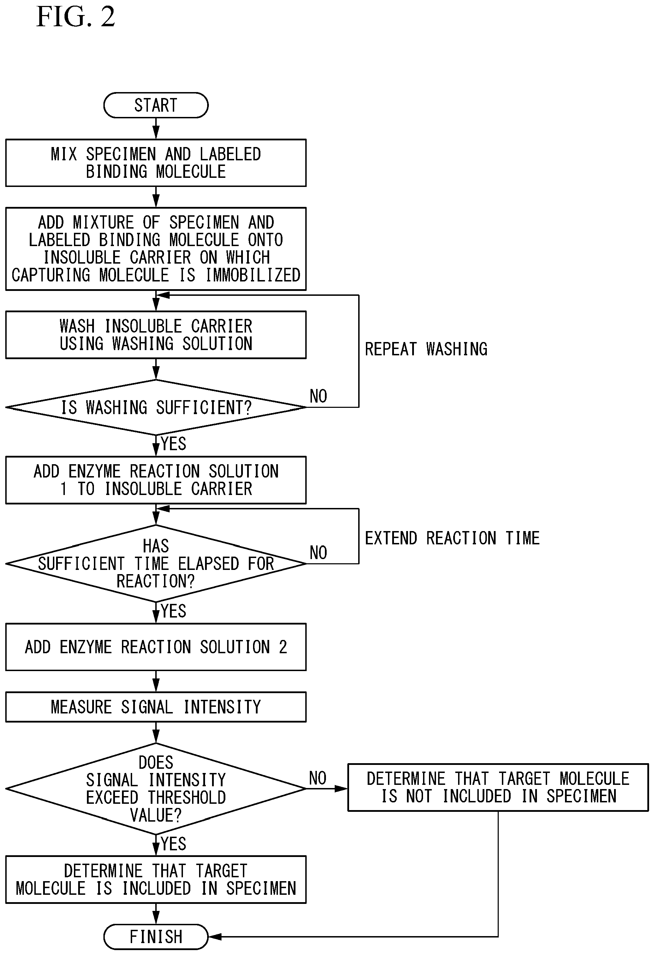

Next, a description will be given of the operating procedure for detecting and quantifying the target molecule by the target molecule detection method of the present embodiment. FIG. 2 is a diagram showing an example of the operating procedure of the target molecule detection method in a case where the ATP amplification step and the ATP detection step are performed separately.

In the target molecule detection method in a case where the ATP amplification step and the ATP detection step are performed separately, first, the specimen and the labeled binding molecules 30 are mixed and the obtained mixture of the specimen and the labeled binding molecules 30 is added onto the insoluble carrier 40 on which the capturing antibody 20 is immobilized. As a result, the (target molecule)-(labeled binding molecule) complex binds to the capturing antibody 20 immobilized on the insoluble carrier 40 and the (target molecule)-(labeled binding molecule)-(capturing molecule) complex is formed on the insoluble carrier 40. Next, the insoluble carrier 40 is washed using a washing solution to remove the labeled binding molecules 30 that did not bind to the target molecules 10. In a case where the washing is insufficient, the washing is repeated. It is possible to determine whether the washing is sufficient, for example, by the presence or absence of labeled binding molecules 30 that did not bind to the target molecules 10 in the washing solution. When there are no more of the labeled binding molecules 30 in the washing solution, it is possible to determine that the washing is sufficient.

When the washing is sufficient, next, an enzyme reaction solution (also referred to below as an enzyme reaction solution 1) including a substrate of the enzyme that catalyzes a reaction to produce ATP, a reagent that amplifies ATP, an enzyme that detects ATP and, as necessary, a cofactor for the enzyme is added to the insoluble carrier 40 and ATP produced by the enzyme that catalyzes a reaction to produce ATP and which is labeled with the labeled binding molecule 30 is amplified by the reagent that amplifies ATP. In a case where a sufficient time has elapsed for the ATP production reaction and the ATP amplification reaction, the process moves on to the ATP detection step. In a case where a sufficient time has not elapsed for the ATP production reaction and the ATP amplification reaction and the amount of ATP amplification is insufficient, the reaction time is extended. It is possible to determine whether or not the amount of ATP amplification is sufficient by whether or not it is possible to detect the amplified ATP.

In a case where the ATP production reaction and amplification reaction time are sufficient and a sufficient amount of ATP is amplified for detection, a substrate (also referred to below as an enzyme reaction solution 2) for detecting ATP is added thereto and the generated signal is measured. For example, when the generated signal is luminescence, the luminescence signal intensity is measured. In a case where the generated signal intensity exceeds a certain threshold value, it is possible to determine that the specimen includes the target molecule 10. In a case where the generated signal intensity does not exceed a certain threshold value, it is possible to determine that the specimen does not include the target molecule 10. It is possible to appropriately set the threshold value of the signal intensity to determine that the specimen includes the target molecule 10 according to the purpose of the detection, detection sensitivity, and the like.

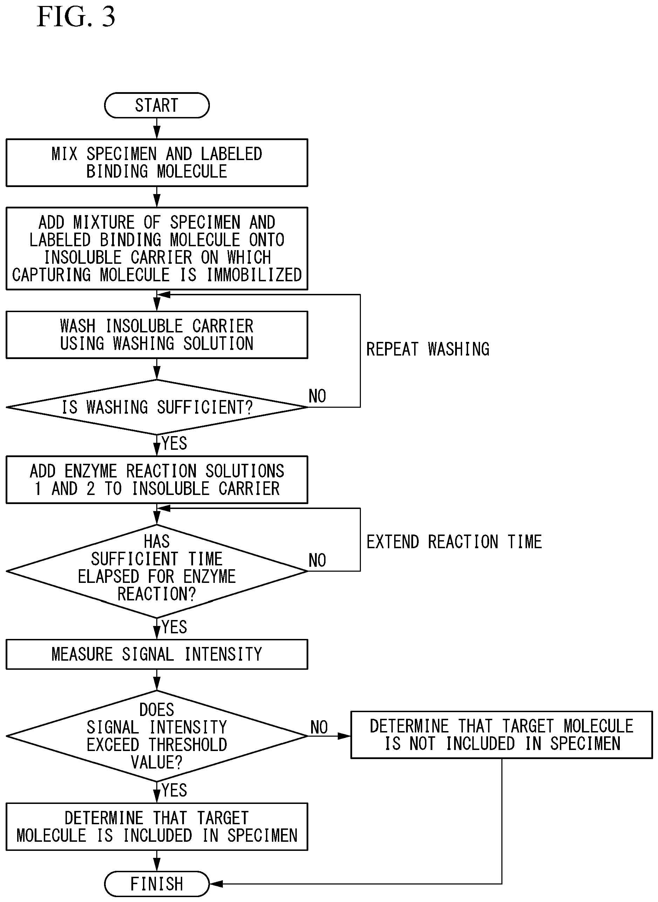

FIG. 3 is a diagram showing an example of an operating procedure of the detection method of a target molecule in a case where the ATP amplification step and detection step are performed simultaneously.

In the detection method of a target molecule in a case where the ATP amplification step and ATP detection step are performed simultaneously, the insoluble carrier 40 is washed using a washing solution and the process is the same as a case where the ATP amplification step and the ATP detection step are performed separately up to the step of removing the labeled binding molecules 30 that did not bind to the target molecules 10. In a case where the ATP amplification step and ATP detection step are performed simultaneously, after washing the insoluble carrier 40, the enzyme reaction solution 1 and the enzyme reaction solution 2 are added to the insoluble carrier 40 and a reaction to amplify the ATP produced by the enzyme that catalyzes a reaction to produce ATP and which is labeled with the labeled binding molecules 30 and a reaction to detect the amplified ATP are performed simultaneously. In a case where a sufficient time has elapsed for the reaction, the signal generated by the reaction is measured. In a case where a sufficient time has not elapsed for the reaction and the amount of ATP detected is insufficient, the reaction time is extended. It is possible to determine whether or not the reaction time is sufficient by the intensity of the signal generated by the reaction.

If the reaction time is sufficient and a signal is generated, the signal intensity is measured. In a case where the generated signal intensity exceeds a certain threshold value, it is possible to determine that the specimen includes the target molecule 10. In a case where the generated signal intensity does not exceed a certain threshold value, it is possible to determine that the specimen does not include the target molecule 10.

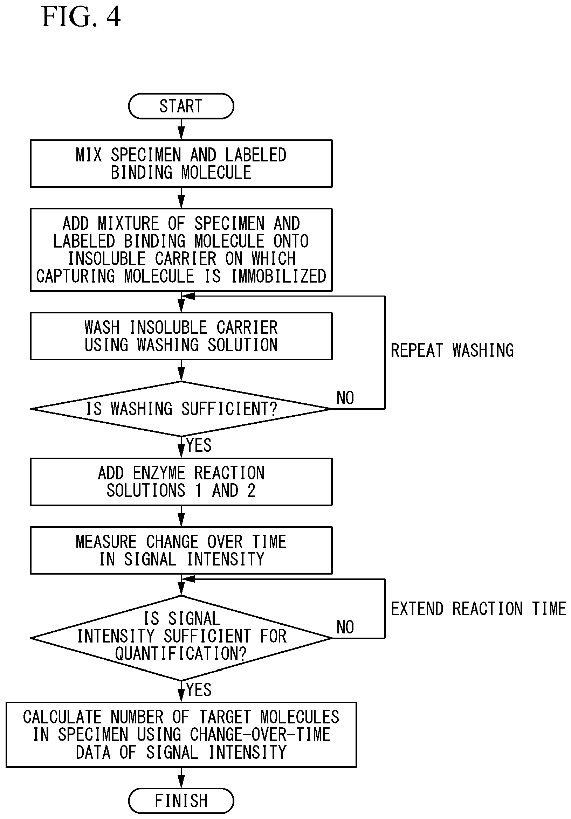

FIG. 4 is a diagram showing an example of the operating procedure of the method for quantifying the target molecules in a case where the ATP amplification step and the ATP detection step are performed simultaneously.

In the method for quantifying the target molecules in a case where the ATP amplification step and the ATP detection step are performed simultaneously, the insoluble carrier 40 is washed with a washing solution and the process is the same as the case of the detection method for target molecules, in which the ATP amplification step and the ATP detection step are performed separately up to the step of removing the labeled binding molecules 30 that did not bind to the target molecules 10. In the case of the method for quantifying the target molecule in which the ATP amplification step and the ATP detection step are performed simultaneously, after washing the insoluble carrier 40, the enzyme reaction solution 1 and the enzyme reaction solution 2 are added to the insoluble carrier 40, the ATP produced by the enzyme that catalyzes a reaction to produce ATP which is labeled with the labeled binding molecule 30 is amplified and, simultaneously, a reaction to detect the amplified ATP is performed, and the change over time in the intensity of the signal generated by the ATP detection reaction is measured from the signal generation start time. In a case where a sufficient time has not elapsed for the reaction and the signal intensity is insufficient for quantification, the reaction time is extended.

In a case where the reaction time is sufficient and the signal intensity is sufficient for quantification, the number of the target molecules included in the specimen is calculated by fitting to signal intensity change-over-time data created in advance by generating a signal in the same manner using a known concentration of the target molecule.

Next, a description will be given of specific examples of immunochromatography using the detection method of a target molecule of the present embodiment. In immunochromatography using the detection method of a target molecule of the present embodiment, it is possible to use apparatuses widely used in ELISA methods.

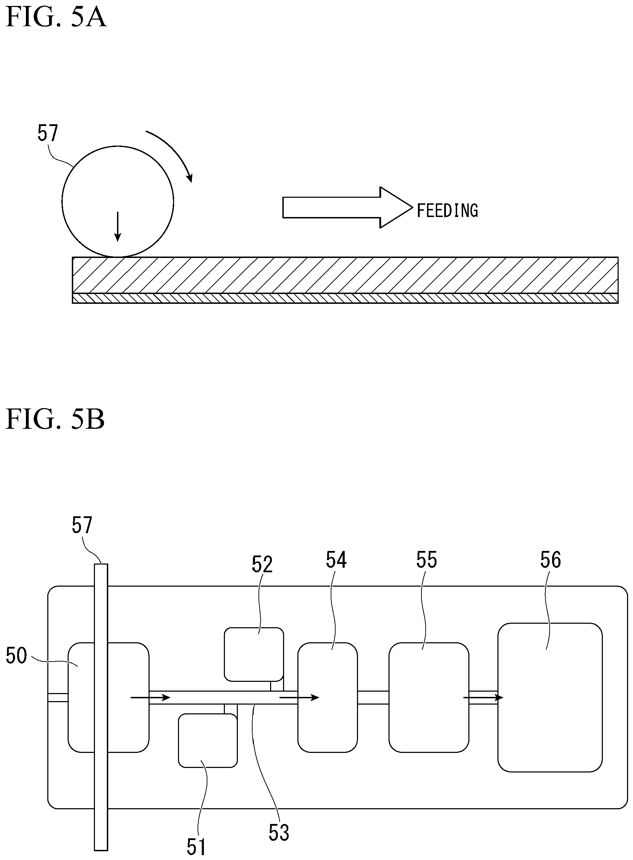

FIG. 5A shows a side surface view of an example configuration in immunochromatography using the detection method of a target molecule of the present embodiment. FIG. 5B shows a top surface view of an example configuration in immunochromatography using the detection method of a target molecule of the present embodiment. The immunochromatography using the detection method of a target molecule of the present embodiment consists of a specimen addition section 50, a first reagent accommodation section 51, a second reagent accommodation section 52, a flow channel 53, a mixing-reaction section 54, a detection section 55, and a waste solution accommodation section 56. The first reagent accommodation section 51 accommodates the labeled binding molecule 30 (also referred to below as a reagent 1). The second reagent accommodation section 52 accommodates a substrate of the enzyme that catalyzes a reaction to produce ATP, a reagent that amplifies ATP, and an enzyme that detects ATP (also referred to below as a reagent 2). The mixing-reaction section 54 accommodates the insoluble carrier 40 on which the capturing molecule 20 is immobilized. It is also possible for the mixing-reaction section 54 to serve as the detection section 55.

First, specimens are added to the specimen addition section 50 using a pipette or the like and fed into the flow channel 53. It is also possible to use automatic feeding using a machine for the specimen feeding. It is also possible to perform the automatic feeding using a cartridge, for example, with a pump or roller that applies force externally to an elastic container to perform the feeding. FIG. 5A and FIG. 5B show an example of feeding using a roller 57.

Next, the reagent 1 accommodated in the first reagent accommodation section 51 is fed by the roller 57 into the flow channel 53, mixed with the specimen to form a (target molecule)-(labeled binding molecule) complex, and supplied to the mixing-reaction section 54. The (target molecule)-(labeled binding molecule) complex supplied to the mixing-reaction section 54 is captured by the capturing molecule 20 immobilized on the insoluble carrier 40 accommodated in the mixing-reaction section 54 and the (target molecule)-(labeled binding molecule)-(capturing molecule) complex is formed on the insoluble carrier 40.

Next, after washing the mixing-reaction section 54 with a washing solution, the reagent 2 accommodated in the second reagent accommodation section 52 is fed by the roller 57 through the flow channel 53 into the mixing-reaction section 54 and added to the (target molecule)-(labeled binding molecule)-(capturing molecule) complex immobilized on the insoluble carrier 40 accommodated in the mixing-reaction section 54 and the ATP is produced and amplified. Next, when the substrate to detect ATP is added to the mixing-reaction section 54, the amplified ATP, the enzyme that detects ATP, and the substrate of the enzyme that detects ATP react to generate a signal. The generated signal is measured by a detector according to the signal in the detection section 55. Possible examples of detectors include plate readers, luminometers, photomultiplier tubes (PMTs), charge-coupled devices (CCDs), CMOS, photosensitive materials (photographic film, instant film, photographic paper, and the like), or the like. After signal measurement, the reaction solution and washing solution are accommodated in the waste solution accommodation section 56 and disposed of.

Next, using FIG. 6, a description will be given of an example of an example configuration and an operation in immunochromatography using each of a virus as the target molecule 10, a PPDK-labeled VHH antibody as the labeled binding substance 30, a DNA aptamer as the capturing antibody 20, a cyclo-olefin polymer (COP) as the insoluble carrier 40, a combination of AK and PK as the enzyme that amplifies ATP, luciferase as the enzyme that detects ATP, and D-luciferin as the substrate that detects ATP, using a cartridge in which feeding is performed by externally applying force to the elastic container with a roller.

### (1) Specimen Addition

A specimen is added to the cartridge from the specimen addition section 50. In FIG. 6, saliva including a virus is shown as an example of a specimen.

### (2) Formation of (Virus)-(PPDK-Labeled VHH Antibody) Complex

The specimen and the PPDK-labeled VHH antibody (the reagent 1) accommodated in the first reagent accommodation section 51 are fed by the roller 57 and the specimen and PPDK-labeled VHH antibody are mixed. The PPDK-labeled VHH antibody forms a complex with the virus in the specimen.

### (3) Capture of (Virus)-(PPDK-Labeled VHH Antibody) Complex by DNA Aptamer

The (virus)-(PPDK-labeled VHH antibody) complex is captured by a DNA aptamer, which is the capturing molecule 20 immobilized on the insoluble carrier 40, and a (virus)-(PPDK-labeled VHH antibody)-(DNA aptamer) complex is formed.

### (4) Washing

A washing solution accommodated in a washing solution accommodation section 58 is fed by the roller 57 and the (virus)-(PPDK-labeled VHH antibody)-(DNA aptamer) complex formed on the COP accommodated in the mixing-reaction section 54 is washed. The washing is performed a plurality of times. This washing removes ATP in the specimen and PPDK-labeled VHH antibodies that did not bind to the virus.

### (5) Addition of Reagent 2

The reagent 2 including phosphoenolpyruvate, pyrophosphate (PPi), AMP, AK, PK, and luciferase (Luc), which is accommodated in the second reagent accommodation section 52, is fed by the roller 57 to the mixing-reaction section 54.

### (6) ATP Production and Amplification

The reagent 2 and the virus-PPDK-labeled VHH antibody-DNA aptamer formed on the COP react to produce ATP and amplify ATP. The result is left to stand until the reaction is sufficiently advanced.

### (7) Addition of Substrate of Enzyme that Detects ATP

D-luciferin, which is a substrate for luciferase, the enzyme that detects ATP, is added to the mixing-reaction section 54. When D-luciferin is added, the luciferase in the reagent 2, the amplified ATP, and the D luciferin react and generate a luminescence signal.

### (8) Detection of Luminescence Signal

The luminescence signal generated in (7) is measured using a detector such as a photomultiplier tube (PMT), a charge-coupled device (CCD), or a CMOS. In a case where the luminescence signal is higher than a preset threshold value, it is possible to determine that a virus is present in the specimen (saliva). In a case where the luminescence signal is lower than the preset threshold value, it is determined that no virus is present in the specimen (saliva).

Next, a description will be given of the detection kit for target molecules of the present embodiment.

The detection kit for target molecules of the present embodiment is a kit used in the detection method of a target molecule of the present embodiment and includes a capturing molecule, a labeled binding molecule, a substrate of the enzyme that catalyzes a reaction to produce ATP, a reagent that amplifies ATP, and a reagent that detects ATP.

In the detection kit for target molecules of the present embodiment, examples of the capturing molecule, the labeled binding molecule, the substrate of the enzyme that catalyzes a reaction to produce ATP, the reagent that amplifies ATP, and the reagent that detects ATP include the capturing molecule, the labeled binding molecule, the substrate of the enzyme that catalyzes a reaction to produce ATP, the reagent that amplifies ATP, and the reagent that detects ATP which were exemplary examples in the detection method of a target molecule of the present embodiment described above, respectively.

The kit of the present embodiment may further include the reagents and apparatus necessary to detect the target molecule by the detection method of a target molecule of the present embodiment.

The kit of the present embodiment may include an insoluble carrier such as a substrate, in addition to the capturing molecule, the labeled binding molecule, the substrate of the enzyme that catalyzes a reaction to produce ATP, the reagent that amplifies ATP, and the reagent that detects ATP. In the case of such a kit, the capturing antibody 20 is immobilized on the insoluble carrier 40 which is a substrate or the like. Examples of the kit including the insoluble carrier 40 on which the capturing antibody 20 is immobilized include, as shown in FIG. 5, a kit including a cartridge formed of the specimen addition section 50, the first reagent accommodation section 51, the second reagent accommodation section 52, the flow channel 53, the mixing-reaction section 54, the detection section 55, and the waste solution accommodation section 56, the labeled binding molecules 30 labeled with an enzyme that catalyzes a reaction to produce ATP, a substrate of the enzyme that catalyzes a reaction to produce ATP, a reagent that amplifies ATP, and a reagent that detects ATP. It is possible for the first reagent accommodation section 51 to accommodate the labeled binding molecule 30 (the reagent 1). It is possible for the second reagent accommodation section 52 to accommodate a reagent (the reagent 2) including a substrate of the enzyme that catalyzes a reaction to produce ATP, a reagent that amplifies ATP, and an enzyme that detects ATP. The mixing-reaction section 54 accommodates the insoluble carrier 40 on which the capturing molecule 20 is immobilized. The first reagent accommodation section 51 and the second reagent accommodation section 52 may accommodate, in advance, the reagent 1 and the reagent 2, respectively.

In such a kit, the specimen is added to the specimen addition section 50 using a pipette or the like and fed into the flow channel 53. It is also possible to use automatic feeding using a machine for the feeding. It is also possible to perform automatic feeding by applying force externally to the elastic container with a pump or roller, for example.

The kit of the present embodiment may further include the necessary buffer solution, enzyme reaction stop solution, detectors such as plate readers and luminometers, product instructions, and the like.

Next, a description will be given of an example of the method for using the kit of the present embodiment.

First, the reagent 1 accommodated in the first reagent accommodation section 51 is fed into the flow channel 53 by the roller 57, mixed with the specimen to form a (target molecule)-(labeled binding molecule) complex, and supplied to the mixing-reaction section 54. The (target molecule)-(labeled binding molecule) complex supplied to the mixing-reaction section 54 is captured by the capturing molecule 20 immobilized on the insoluble carrier 40 accommodated in the mixing-reaction section 54 and the (target molecule)-(labeled binding molecule)-(capturing molecule) complex is formed on the insoluble carrier 40.

Next, after the mixing-reaction section 54 is washed with a washing solution, the reagent 2 accommodated in the second reagent accommodation section 52 is fed into the mixing-reaction section 54 through the flow channel 53 by the roller 57. Next, the reagent 2 is added to the (target molecule)-(labeled binding molecule)-(capturing molecule) complex formed on the insoluble carrier 40 accommodated in the mixing-reaction section 54 to produce and amplify ATP. Next, a substrate to detect ATP is added to the mixing-reaction section 54 and the amplified ATP, the enzyme that detects ATP, and the substrate of the enzyme that detects ATP are reacted to generate a signal. The generated signal is measured by a detector according to the signal in the detection section 55. Possible examples of detectors include plate readers, luminometers, and the like. After the signal measurement, the reaction solution and washing solution are accommodated in the waste solution accommodation section 56 and disposed of.

The application scope of the present invention is not limited to the embodiments described above. It is possible to widely apply the present invention to methods and kits for detecting target molecules included in specimens by immunoassay methods.

### [Examples]

A more detailed description will be given of the present invention on the basis of Examples, but the present invention is not limited thereto.

### (Example 1)

Signal intensities were compared for a case where the enzyme that catalyzes a reaction to produce ATP was used alone and a case where the enzyme that catalyzes a reaction to produce ATP was used in combination with a reagent that amplifies ATP. PPDK was used as the enzyme that catalyzes a reaction to produce ATP. AK and PK were used as enzymes that amplify ATP. Beetle luciferase and a substrate thereof were used as reagents that detect ATP. The reagents used in these Examples are given below.

- Pyruvate phosphate dikinase (PPDK, manufactured by BioVision, Inc.)
- Adenylate kinase (AK, manufactured by Sigma)
- Pyruvate kinase (PK, manufactured by Sigma)
- Beetle luciferase (Luc, manufactured by Sigma)
- AMP (manufactured by Fujifilm Wako Pure Chemical Corp.)
- Pyrophosphate (PPi, manufactured by Fujifilm Wako Pure Chemical Corp.)
- D-luciferin (manufactured by Fujifilm Wako Pure Chemical Corp.)
- Phosphoenolpyruvate (PEP, manufactured by Fujifilm Wako Pure Chemical Corp.)
- ATP (manufactured by Fujifilm Wako Pure Chemical Corp.)
- MgCl₂ (manufactured by Fujifilm Wako Pure Chemical Corp.)

Next, these reagents were dissolved in PBS (pH 7.4) to the final concentrations shown in Table 1 below and samples with the sample numbers 1 to 7 were prepared.

**Table 1**

| Sample No. | Reagent | | | | | | | | |
|---|---|---|---|---|---|---|---|---|---|
| 1 | | 10 µg/ml PPDK | 1 mM PPi | 1 mM PEP | 1 mM AMP | 10 µg/ml PK | 10 µg/ml AK | 10 µg/ml Luc | 10 mM MgCl₂ |
| 2 | | 2 µg/ml PPDK | 1 mM PPi | 1 mM PEP | 1 mM AMP | 10 µg/ml PK | 10 µg/ml AK | 10 µg/ml Luc | 10 mM MgCl₂ |
| 3 | 20 µM ATP | | 1 mM PPi | 1 mM PEP | 1 mM AMP | 10 µg/ml PK | 10 µg/ml AK | 10 µg/ml Luc | 10 mM MgCl₂ |
| 4 | | 10 µg/ml PPDK | 1 mM PPi | 1 mM PEP | 1 mM AMP | | | 10 µg/ml Luc | 10 mM MgCl₂ |
| 5 | | 2 µg/ml PPDK | 1 mM PPi | 1 mM PEP | 1 mM AMP | | | 10 µg/ml Luc | 10 mM MgCl₂ |
| 6 | 20 µM ATP | | | | | | | 10 µg/ml Luc | 10 mM MgCl₂ |
| 7 | | | 1 mM PPi | 1 mM PEP | 1 mM AMP | | | 10 µg/ml Luc | 10 mM MgCl₂ |

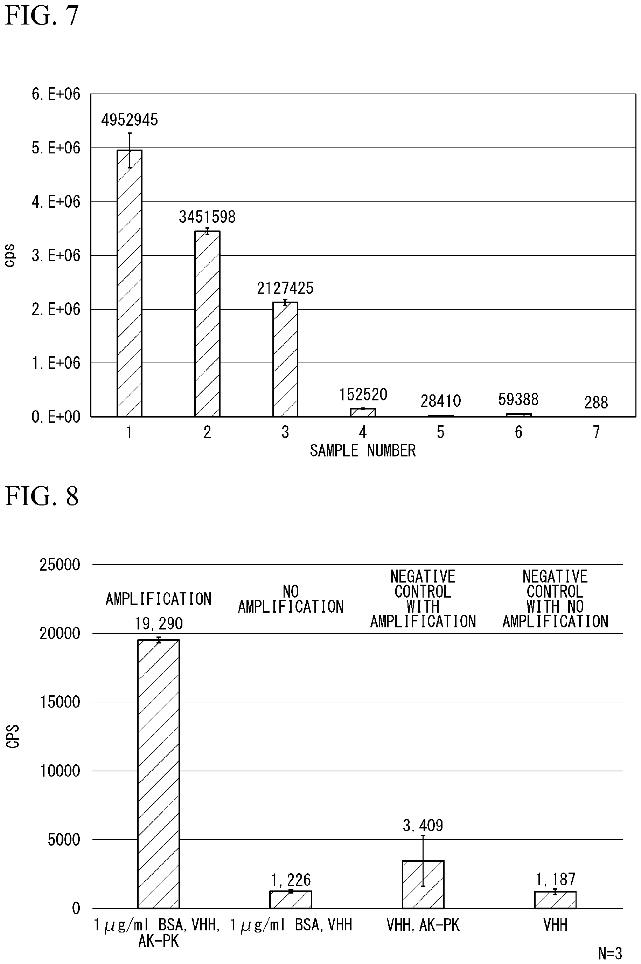

Each sample was prepared so as to be 50 µl in each well of a 96-well plate (manufactured by PerkinElmer, Inc.) (N=4). After sample preparation, the samples were incubated at room temperature for 10 minutes to allow the ATP production reaction and ATP amplification reaction to proceed. After incubation, D-luciferin was added to a final concentration of 2 mM and the luminescence intensity of each sample was measured using an EnVision multimode plate reader (manufactured by PerkinElmer, Inc.). The luminescence intensity was measured by a method for measuring the amount of luminescence per second (counts per second; cps) per well of a 96-well plate. The results are shown in FIG. 7.

As shown in FIG. 7, the average values of the luminescence intensity for the samples with sample number 4 and sample number 5 in cases where PPDK was used alone and an ATP amplification reaction using AK and PK was not performed were 152,520 and 28,410. In contrast, the average values of the luminescence intensity for the samples with sample number 1 and sample number 2 in cases in which PPDK was used in combination with an ATP amplification reaction using AK and PK were 4,952,945 and 3,451,598. The luminescence intensity exhibited by the sample with sample number 1 was 32.5 times higher than that of the sample with sample number 4 and that of the sample with sample number 2 was 121.5 times higher than that of the sample with sample number 5, indicating that, under a condition of a lower concentration of PPDK, the combination of the ATP production reaction and the ATP amplification reaction was advantageous for signal enhancement.

From the above, compared to the method using the enzyme that catalyzes a reaction to produce ATP alone, it was shown that the method of the present invention using the combination of the enzyme that catalyzes a reaction to produce ATP and the reagent that amplifies ATP has a remarkable effect in detecting ATP. Furthermore, the signal enhancement efficiency increases in a case where the amount of PPDK, which is the enzyme that catalyzes a reaction to produce ATP, is low, thus, even in a case where the amount of labeled binding molecules binding to the target molecule is low, that is, in a case where there are few target molecules, it is considered that the target molecule detection method of the present invention is capable of detecting the target molecule.

### (Example 2)

Using a PPDK-labeled anti-BSA-camelid heavy chain variable region antibody (also referred to below as PPDK-labeled anti-BSA-VHH antibody), the signal intensities were compared when performing BSA detection for a case where a PPDK-labeled anti-BSA-VHH antibody was used alone and for a case where a PPDK-labeled anti-BSA-VHH antibody and a reagent that amplifies ATP were used in combination. AK and PK were used as enzymes to amplify ATP. Beetle luciferase and a substrate thereof were used as reagents that detect BSA. The reagents used in these Examples are given below.

- PPDK-labeled anti-BSA-VHH antibody (manufactured by RePHAGEN Co., Ltd.)
- Biotin-labeled anti-BSA antibody (manufactured by ITEA)
- Adenylate kinase (AK, manufactured by Nipro)
- Pyruvate kinase (PK, manufactured by Nipro)
- Beetle luciferase (Luc, manufactured by Sigma)
- AMP (manufactured by Fujifilm Wako Pure Chemical Corp.)
- Pyrophosphate (PPi, manufactured by Fujifilm Wako Pure Chemical Corp.)
- D-luciferin (manufactured by Fujifilm Wako Pure Chemical Corp.)
- Phosphoenolpyruvate (PEP, manufactured by Fujifilm Wako Pure Chemical Corp.)
- MgCl₂ (manufactured by Fujifilm Wako Pure Chemical Corp.)

Using the reagents described above, BSA was detected by the following procedure.

### (1) Plate Preparation

A Nunc Immobilizer Streptavidin F96 Black plate (manufactured by Thermo) was washed three times with 300 µl of PBS using a plate washer Wellwash Versa (manufactured by Thermo). Next, 100 µl each of biotin-labeled anti-BSA antibodies adjusted to 1 µg/ml was added to each well and incubated at room temperature for 1 hour. After removing the biotin-labeled anti-BSA antibody solution in each well, washing was carried out three times with 300 µl of PBS using a plate washer.

### (2) Addition of Samples

50 µl each of a 1 µg/ml BSA solution was added to each well (N=3) and incubated at room temperature for 1 hour. In addition, 50 µl of PBS (control) was also added to each well (N=3) in the same manner. After removing the BSA solution or PBS from each well, each well was washed three times with 300 µl of PBS. Next, 50 µl of PPDK-labeled anti-BSA-VHH antibody solution diluted in 1 µg/ml with PBS was added to each well and incubated at room temperature for 1 hour. After removing the PPDK-labeled anti-BSA-VHH antibody solution from each well, each well was washed four times with 300 µl of PBS using a plate washer.

### (3) Signal Detection

After adding 5 µl of AK adjusted to a final concentration of 10 µg/ml to each well, 10 µl each of a PK enzyme solution adjusted to a final concentration of 10 µg/ml was added thereto. Next, after 10 µl of PBS was added to each well, 25 µl of a reagent A (PBS including 1 mM PPi, 1 mM PEP, 1 mM AMP, and 10 mM Mg Cl₂) was added with an 8-channel pipettor.

The 96-well plate obtained as described above was then set in a luminometer GloMax navigator (manufactured by Promega) and allowed to stand for 15 minutes. 50 µl of a reagent B (PBS including 5 µg/ml Luc and 200 µM D-luciferin) was added to each well using the pump of a luminometer and the luminescence intensity of each sample was measured immediately after addition. The luminescence intensity was measured by a method for measuring the amount of luminescence per second (counts per second; cps) per well in each 96-well plate. The measurement results are shown in FIG. 8.

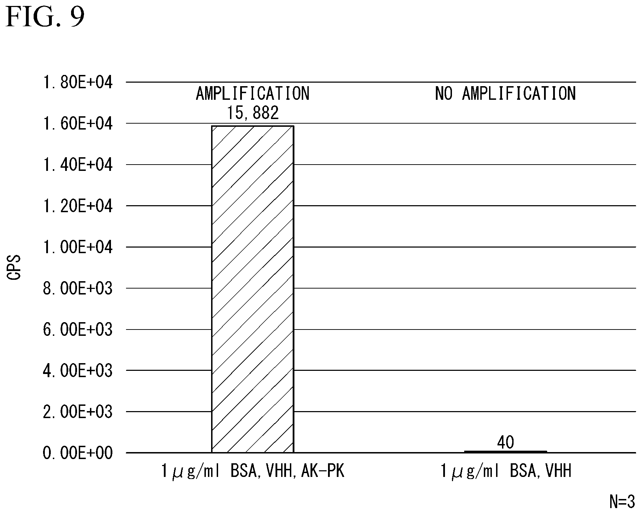

As shown in FIG. 8, the luminescence intensity in a case where the PPDK-labeled anti-BSA-VHH antibody was used alone was an average of 1226 cps and the difference was slight compared to the luminescence intensity of 1187 cps of the negative control in a case where ATP amplification was not performed. On the other hand, the luminescence intensity in a case where the PPDK-labeled anti-BSA-VHH antibody was used in combination with a reagent that amplifies ATP was 19,290 cps, and compared to the luminescence intensity of 3409 cps of the negative control in a case where the ATP amplification was performed, the luminescence intensity was greatly amplified. For a case where a PPDK-labeled anti-BSA-VHH antibody was used in combination with a reagent that amplifies ATP and a case where a PPDK-labeled anti-BSA-VHH antibody was used alone, FIG. 9 shows a graph of luminescence intensity in which the luminescence intensity of the negative control in a case where ATP amplification was performed and the luminescence intensity of the negative control in a case where ATP amplification was not performed, are each subtracted as a background.

As shown in FIG. 9, in comparison with a case where the PPDK-labeled anti-BSA-VHH antibody is used alone and ATP amplification is not performed, it is clear that a case where the PPDK-labeled anti-BSA-VHH antibody is combined with reagents that amplify ATP and ATP amplification was performed had a greatly increased luminescence intensity. The above indicates that the method of the present invention has a remarkable effect in detecting target molecules included in a sample. Even in a case where signal detection is difficult in a case where there are few target molecules and antibodies labeled with an ATP-producing enzyme are used alone, it is considered that the detection method of a target molecule of the present invention is capable of detecting target molecules by amplifying the signal.

### [Reference Signs List]

10: Target molecule
20: Capturing molecule
30: Labeled binding molecule
40: Insoluble carrier
50: Specimen addition section
51: First reagent section
52: Second reagent section
53: Flow path
54: Mixing-reaction section
55: Detection section
56: Waste solution accommodation section
57: Roller
58: Washing solution accommodation section

## Claims

1. A detection method of a target molecule in a specimen, the method comprising:
step (1): a complex formed of a target molecule, a labeled binding molecule, and a capturing molecule (referred to below as a (target molecule)-(labeled binding molecule)-(capturing molecule) complex) forming step of reacting the target molecule in the specimen, a capturing molecule that binds to the target molecule, and a labeled binding molecule that binds to the target molecule and which is labeled with an enzyme that catalyzes a reaction to produce ATP, to form a (target molecule)-(labeled binding molecule)-(capturing molecule) complex;
step (2): a step of removing the labeled binding molecules which did not bind to the target molecule in the step (1);
step (3): an ATP production step of producing ATP by reacting the (target molecule)-(labeled binding molecule)-(capturing molecule) complex with a substrate of the enzyme that catalyzes a reaction to produce ATP;
step (4): an ATP amplification step of amplifying the ATP produced in the step (3); and
step (5): an ATP detection step of detecting the ATP amplified in the step (4).

2. The detection method according to Claim 1, further comprising:
a step of removing ATP in the specimen in advance before forming the (target molecule)-(labeled binding molecule)-(capturing molecule) complex in the step (1).

3. The detection method according to Claim 1 or 2,
wherein the capturing molecule is an antibody or antibody fragment, or an aptamer that specifically binds to the target molecule.

4. The detection method according to any one of Claims 1 to 3,
wherein the labeled binding molecule is an antibody or antibody fragment, or an aptamer that specifically binds to the target molecule and which is labeled with the enzyme that catalyzes a reaction to produce ATP.

5. The detection method according to any one of Claims 1 to 4,
wherein the enzyme that catalyzes a reaction to produce ATP is pyruvate phosphate dikinase, and the substrates of the enzyme that catalyzes a reaction to produce ATP are phosphoenolpyruvate, pyrophosphate, and AMP.

6. The detection method according to any one of Claims 1 to 4,
wherein the enzyme that catalyzes a reaction to produce ATP is acetyl-CoA synthetase, and the substrates of the enzyme that catalyzes a reaction to produce ATP are AMP, pyrophosphate, and acetyl-CoA.

7. The detection method according to any one of Claims 1 to 4,
wherein the enzyme that catalyzes a reaction to produce ATP is ATP-sulfurylase, and the substrates of the enzyme that catalyzes a reaction to produce ATP are adenosine 5'-phosphosulfate and pyrophosphate.

8. The detection method according to any one of Claims 1 to 4,
wherein the enzyme that catalyzes a reaction to produce ATP is a type 11 polyphosphate kinase, and the substrates of the enzyme that catalyzes a reaction to produce ATP are AMP and polyphosphate.

9. The detection method according to any one of Claims 1 to 8,
wherein the amplification of the ATP is performed using adenylate kinase and pyruvate kinase in the step (4).

10. The detection method according to any one of Claims 1 to 8,
wherein the amplification of the ATP is performed using adenylate kinase and polyphosphate kinase in the step (4).

11. The detection method according to any one of Claims 1 to 8,
wherein the amplification of the ATP is performed using adenylate kinase and acetate kinase in the step (4).

12. The detection method according to any one of Claims 1 to 11,
wherein the detection of the ATP is performed using luciferase in the step (5).

13. A detection kit for a target molecule in a specimen, the kit comprising:
an insoluble carrier on which a capturing molecule that binds to the target molecule is immobilized;
a labeled binding molecule that binds to the target molecule and which is labeled with an enzyme that catalyzes a reaction to produce ATP;
a substrate of the enzyme that catalyzes a reaction to produce ATP;
a reagent that amplifies ATP; and
a reagent that detects ATP.

14. The detection kit according to Claim 13,
wherein the capturing molecule is an antibody or antibody fragment, or an aptamer that specifically binds to the target molecule.

15. The detection kit according to Claim 13 or 14,
wherein the labeled binding molecule is an antibody or antibody fragment, or an aptamer that specifically binds to the target molecule and which is labeled with the enzyme that catalyzes a reaction to produce ATP.

16. The detection kit according to any one of Claims 13 to 15,
wherein the enzyme that catalyzes a reaction to produce ATP is pyruvate phosphate dikinase, and the substrates of the enzyme that catalyzes a reaction to produce ATP are phosphoenolpyruvate, pyrophosphate, and AMP.

17. The detection kit according to any one of Claims 13 to 15,
wherein the enzyme that catalyzes a reaction to produce ATP is acetyl-CoA synthetase, and the substrates of the enzyme that catalyzes a reaction to produce ATP are AMP, pyrophosphate, and acetyl-CoA.

18. The detection kit according to any one of Claims 13 to 15,
wherein the enzyme that catalyzes a reaction to produce ATP is ATP-sulfurylase, and the substrates of the enzyme that catalyzes a reaction to produce ATP are adenosine 5'-phosphosulfate and pyrophosphate.

19. The detection kit according to any one of Claims 13 to 15,
wherein the enzyme that catalyzes a reaction to produce ATP is a type II polyphosphate kinase, and the substrates of the enzyme that catalyzes a reaction to produce ATP are AMP and polyphosphate.

20. The detection kit according to any one of Claims 13 to 19,
wherein the reagent that amplifies ATP includes AMP, phosphoenolpyruvate, magnesium ions, adenylate kinase, and pyruvate kinase.

21. The detection kit according to any one of Claims 13 to 19,
wherein the reagent that amplifies ATP includes AMP, polyphosphate, magnesium ions, adenylate kinase, and polyphosphate kinase.

22. The detection kit according to any one of Claims 13 to 19,
wherein the reagent that amplifies ATP includes AMP, acetyl phosphate, magnesium ions, adenylate kinase, and acetate kinase.

23. The detection kit according to any one of Claims 13 to 22,
wherein the reagent that detects ATP includes D-luciferin and luciferase.
